(19) **Europäisches Patentamt / European Patent Office / Office européen des brevets**

(11) **EP 4 769 081 A1**

(12) **DEMANDE DE BREVET EUROPEEN**

(43) Date de publication:
01.07.2026 Bulletin 2026/27

(21) Numéro de dépôt: 25227309.9

(22) Date de dépôt: 26.12.2025

(51) Classification Internationale des Brevets (IPC):
$G06F\ 3/01^{(2006.01)}$    $A61B\ 5/369^{(2021.01)}$
$A61B\ 5/372^{(2021.01)}$    $A61B\ 5/00^{(2006.01)}$
$A61B\ 5/37^{(2021.01)}$

(52) Classification Coopérative des Brevets (CPC):
G06F 3/015; A61B 5/37; A61B 5/726; A61B 5/7267

(84) Etats contractants désignés:
AL AT BE BG CH CY CZ DE DK EE ES FI FR GB GR HR HU IE IS IT LI LT LU LV MC ME MK MT NL NO PL PT RO RS SE SI SK SM TR
Etats d'extension désignés:
BA
Etats de validation désignés:
GE KH LA MA MD TN

(30) Priorité: 27.12.2024 FR 2415310

(71) Demandeur: Commissariat à l'Energie Atomique et aux Energies Alternatives
75015 Paris (FR)

(72) Inventeurs:
• SOURIAU, Rémi
38054 GRENOBLE CEDEX 09 (FR)
• MARTEL, Félix
38054 GRENOBLE CEDEX 09 (FR)
• AKSENOVA, Tétiana
38054 GRENOBLE CEDEX 09 (FR)

(74) Mandataire: INNOV-GROUP
209 Avenue Berthelot
69007 Lyon (FR)

(54) **PROCÉDÉ D'APPRENTISSAGE EN LIGNE D'UNE INTERFACE NEURONALE**

(57) Procédé d'apprentissage d'une interface neuronale directe, l'interface neuronale directe étant reliée à des capteurs ($2_1...2_{I1}$) préalablement disposés autour du cerveau d'un utilisateur, l'interface étant configurée pour commander un actionneur (6) en fonction de signaux électrophysiologiques détectés par chaque capteur, le procédé d'apprentissage comportant une formation d'un modèle prédictif (F), par régression entre un tenseur d'apprentissage, formé ) partir des signaux électrophysiologiques détectés et un tenseur de commande, représentatif de tâches mentales exécutées par l'utilisateur durant différentes époques temporelles, le modèle prédictif permettant d'estimer une tâche, imaginée par l'utilisateur en fonction des signaux électrophysiologiques détectés à chaque époque, le procédé étant tel que la formation du modèle direct est effectuée en fonction du poids respectivement assigné à chaque époque.

Fig. 2

**Description**

## DOMAINE TECHNIQUE

Le domaine technique de l'invention concerne les interfaces neuronales directes, usuellement désignées par le terme « BCI » (Brain Computer Interface), destinées à commander, à partir de signaux neurophysiologiques, un actionneur.

## ART ANTERIEUR

Le domaine des interfaces neuronales directes est en plein développement et apparaît comme une solution séduisante pour permettre à des utilisateurs en situation de handicap de commander des actionneurs par la pensée. Il s'agit de détecter et d'enregistrer des signaux électrophysiologiques émis par le cortex. Ces derniers sont traités par des algorithmes permettant de former un signal de commande, pour commander des actionneurs. Le signal de commande permet le pilotage de l'actionneur, ce dernier étant un exosquelette, un ordinateur ou d'un robot, pour fournir une assistance à l'utilisateur. Les algorithmes mis en œuvre permettent une traduction d'une instruction donnée par l'utilisateur, cette instruction étant captée, par des électrodes, sous la forme des signaux dits électrophysiologiques, car représentatifs de l'activité électrique des neurones. Cette activité électrique peut être mesurée au niveau du cortex, au moyen d'électrodes corticales disposées dans la boîte crânienne. Elle peut également être mesurée par des électrodes d'électroencéphalographie, moins intrusives car disposées sur le scalp, mais également moins performantes, notamment au niveau de la résolution spatiale. Une autre solution est d'enregistrer les signaux électrophysiologiques par magnétoencéphalographie, ce qui nécessite une installation dédiée.

Les algorithmes mis en œuvre sont généralement basés sur un modèle prédictif. Le modèle prédictif utilise des données d'entrée, obtenues par un prétraitement des signaux électrophysiologiques enregistrés, pour établir un signal de commande, à destination de l'actionneur ou des actionneurs. Le signal de commande doit correspondre à une intention exprimée par l'utilisateur, dont on enregistre les signaux électrophysiologiques. L'intention exprimée par l'utilisateur se manifeste sous la forme des signaux électrophysiologiques, ces derniers étant enregistrés et transmis vers l'interface neuronale directe, formant des données d'observations. Les signaux électrophysiologiques sont traités pour obtenir des données d'observations, formant des données d'entrée du modèle prédictif, ce dernier générant un signal de commande correspondant à l'intention exprimée par l'utilisateur. Le signal de commande permet une commande de l'actionneur.

Les signaux mesurés sont traitées pour former des données d'observation généralement multidimensionnelles, et comprennent :

- une composante spatiale, représentative de l'origine spatiale du signal électrophysiologique ;
- une composante fréquentielle, représentative de l'intensité du signal électrophysiologique dans différentes bandes de fréquence ;
- une composante temporelle.

Chaque donnée d'observation est associée à une époque, c'est-à-dire à un intervalle temporel de durée prédéterminée, par exemple de l'ordre de 1 seconde après que l'utilisateur ait l'intention d'effectuer la tâche. A chaque époque, on forme un tenseur d'observation, rassemblant les données d'observation. A partir du tenseur d'observation, on alimente un modèle prédictif. Le modèle prédictif, appliqué au tenseur d'observation, permet d'estimer un signal de commande, permettant la commande des actionneurs. Le signal de commande est généralement exprimé par un vecteur de commande.

Le modèle prédictif est établi au cours d'une phase d'apprentissage, au cours de laquelle l'utilisateur effectue des tâches prédéfinies, pour lesquelles la sortie du modèle prédictif est connue. L'objectif est alors, suite à chaque tâche, de déterminer des composantes des signaux électrophysiologiques enregistrés spécifiques de la tâche. Il peut notamment s'agir de déterminer des corrélations entre des composantes des signaux électrophysiologiques et la sortie du modèle.

L'établissement de modèles prédictifs a amplement été décrit. Par exemple, le brevet US9480583 décrit l'application d'une méthode de régression linéaire des moindres carrés partiels multivariée permettant d'établir un modèle prédictif. Une telle méthode est connue sous l'acronyme anglosaxon "NPLS" ou par le terme "N-way Partial Least Squares". L'application d'une telle méthode a également été décrite dans la publication Eliseyev A, Aksenova T (2013) "Recursive N-way Partial Least Squares for Brain Computer Interface" PIOS ONE july 2013, Volume 8 Issue 7 e69962. Une telle méthode est également décrite dans le document Yelisyeyev A « Brain-Computer Interface with cortical electrical activity recording" Human health and pathology ». Université de Grenoble, 2011.

Cependant, le recours à une méthode de type NPLS nécessite de traiter un grand nombre de données d'apprentissage, par exemple plusieurs centaines ou plusieurs milliers pour une sortie du modèle correspondant à une tâche spécifique. Cela suppose un stockage en mémoire d'une quantité d'information élevée, ce qui n'est pas approprié pour effectuer un apprentissage en ligne, c'est-à-dire en temps réel, ou en quasi temps réel. Par quasi temps réel, on entend un apprentissage effectué par séquences successives, chaque séquence durant quelques secondes ou quelques minutes.

Afin de diminuer la quantité d'information à mémoriser, un procédé d'apprentissage mettant en œuvre une méthode de type REW-NPLS a été développée, REW signifiant « Recursive Exponentially Weighted », ou pondération récursive exponentielle. La formation d'un modèle prédictif, par REW-NPLS, appliqué à une interface BCI, est décrit dans EP3563218. Une telle approche est également justifiée par le fait que les signaux neuronaux ne sont pas stationnaires, ce qui impose une mise à jour régulière du modèle prédictif.

Les inventeurs proposent une amélioration de la méthode décrite dans EP3563218, de façon à améliorer la performance d'apprentissage du modèle prédictif.

## EXPOSE DE L'INVENTION

Un premier objet de l'invention est un procédé d'apprentissage d'une interface neuronale directe, l'interface neuronale directe étant reliée à des capteurs préalablement disposés autour du cerveau d'un utilisateur, chaque capteur étant configuré pour détecter un signal électrophysiologique dépendant d'une activité neuronale de l'utilisateur, l'interface étant configurée pour commander un actionneur en fonction des signaux électrophysiologiques détectés, le procédé d'apprentissage comportant :

- a) sélection d'une tâche mentale à effectuer, choisie parmi une liste de tâches prédéterminée ;
- b) exécution, par l'utilisateur, de la tâche mentale sélectionnée lors de l'étape a) et, durant l'exécution de la tâche,

  • acquisition des signaux électroniques, issus des différents capteurs ;
  • extraction de caractéristiques des signaux électroniques ;
  • formation d'un tenseur d'observation à partir des caractéristiques des signaux extraites ;

- c) réitération des étapes a) et b) durant un nombre prédéterminé d'époques temporelles formant une séquence;
- d) formation d'un tenseur d'apprentissage à partir des tenseurs d'observation formés à chaque époque temporelle, et d'un tenseur de commande à partir des tâches sélectionnées à chaque époque, chaque terme du tenseur d'apprentissage et du tenseur de commande étant associés à une époque de la séquence ;
- e) formation d'un modèle prédictif, par régression entre le tenseur d'apprentissage et le tenseur de commande, le modèle prédictif permettant d'estimer une tâche, choisie parmi la liste des tâches, imaginée par l'utilisateur en fonction du tenseur d'observation formé à chaque époque ;
  les étapes b), d) et e) étant mises en œuvre par une unité de traitement ;

le procédé étant caractérisé en ce qu'il comporte :

- définition d'un critère de pondération pour chaque époque ;
- assignation d'un poids à chaque époque, le poids étant défini en fonction du critère de pondération pour ladite époque, en fonction duquel à deux époques différentes, de la séquence, pour lesquelles le critère de pondération est différent sont assignés deux poids différents ;

le procédé étant tel que la formation du modèle direct est effectuée en fonction du poids respectivement assigné à chaque époque.

Le terme époque correspond à l'équivalent du terme anglosaxon « epoch », et correspond à un plage temporelle, de durée prédéterminée, par exemple 1 seconde, au cours de laquelle les étapes a) et b) sont mises en œuvre.

Le poids assigné à une époque peut dépendre de la tâche sélectionnée durant ladite époque. Selon une possibilité :

- les étapes a) à e) sont mises en œuvre durant plusieurs séquences successives ;
- le critère de pondération est une fréquence d'occurrence de chaque tâche, le poids de chaque époque est d'autant plus élevé que le nombre d'occurrences de la tâche, suite aux séquences successives effectuées, est faible.

Selon une possibilité, après chaque nouvelle séquence, le procédé comporte une mise à jour d'un nombre total d'occurrences pondéré pour chaque tâche, la mise à jour comportant, pour chaque tâche :

- détermination d'un nombre d'occurrences dans la nouvelle séquence ;
- pondération du nombre d'occurrences, dans la nouvelle séquence, par le poids respectivement assigné à la tâche dans la nouvelle séquence ;
- sommation du nombre d'occurrences pondéré de la tâche, dans la nouvelle séquence, au nombre total pondéré pour ladite tâche résultant de la séquence précédent, ce dernier étant multiplié par un facteur d'oubli.

Selon une possibilité, le critère de pondération est une performance d'apprentissage, le procédé comportant :

- détermination d'un indicateur de performance d'apprentissage de chaque tâche suite à chaque époque ;
- détermination du poids de chaque tâche en fonction du critère de performance d'apprentissage de la tâche.

Selon une possibilité, le critère de pondération est une qualité des signaux collectés à chaque séquence, le procédé comportant :

- détermination d'un critère de qualité des signaux collectés à chaque séquence ;
- détermination du poids de chaque tâche en fonction du critère de qualité des signaux collectés respectivement durant l'exécution de chaque tâche.

Selon une possibilité, dans l'étape e), le modèle prédictif est formé par régression multivariée, comportant un calcul d'un tenseur de covariance croisée entre le tenseur d'apprentissage et le tenseur de commande, le tenseur de covariance croisé de chaque séquence étant établi à partir d'un produit :

- du tenseur d'observation;
- du tenseur de commande;
- des poids respectivement assignés à chaque époque.

Selon une possibilité :

- l'étape c) est répétée de façon à former plusieurs séquences successives, à chaque séquence étant assigné un rang ;
- l'étape d) est mise en œuvre pour chaque séquence ;
- lors de l'étape e), le modèle prédictif est formé à partir de deux séquences successives, à partir d'une somme du tenseur de covariance croisée établi pour la séquence de rang supérieur et du tenseur de covariance croisée établi pour la séquence de rang inférieur multiplié par un facteur d'oubli.

Selon une possibilité :

- le tenseur d'apprentissage et le tenseur de commande sont formés d'une matrice, dont une dimension est le nombre d'époques dans la séquence ;
- les poids assignés à chaque époque de la séquence forment une matrice diagonale dont chaque dimension est le nombre d'époques par séquence, chaque terme de la matrice diagonale correspondant au poids assigné à chaque époque respectivement exécutée lors de ladite séquence.

Selon une possibilité, pour au moins une tâche spécifique, le poids est déterminé de façon que le nombre d'occurrences de ladite tâche spécifique, pondéré par le poids assigné à la tâche spécifique, soit supérieur au nombre d'occurrence d'au moins une autre tâche, pondéré par le poids assigné à ladite autre tâche.

Selon une possibilité, le poids assigné à chaque tâche est borné par une valeur maximale prédéfinie.

Un autre objet de l'invention est une interface neuronale directe, l'interface neuronale directe comportant des capteurs préalablement disposés autour du cerveau d'un utilisateur, et configurés pour détecter des signaux électrophysiologiques, représentatifs d'une activité neuronale de l'utilisateur, l'interface étant programmée pour commander un actionneur, en mettant en œuvre un modèle prédictif de commande, le modèle prédictif étant configuré pour générer un signal de commande de l'actionneur à partir de signaux électrophysiologiques détectés, l'interface comportant une unité de traitement, configurée pour acquérir les signaux électroniques lors de chaque étape b), et pour mettre en œuvre les étapes d) et e) d'un procédé selon le premier objet de l'invention.

L'actionneur peut être un dispositif externe à l'utilisateur ou un dispositif implanté dans le corps de l'utilisateur.

L'invention sera mieux comprise à la lecture de l'exposé des exemples de réalisation présentés, dans la suite de la description, en lien avec les figures listées ci-dessous.

**FIGURES**

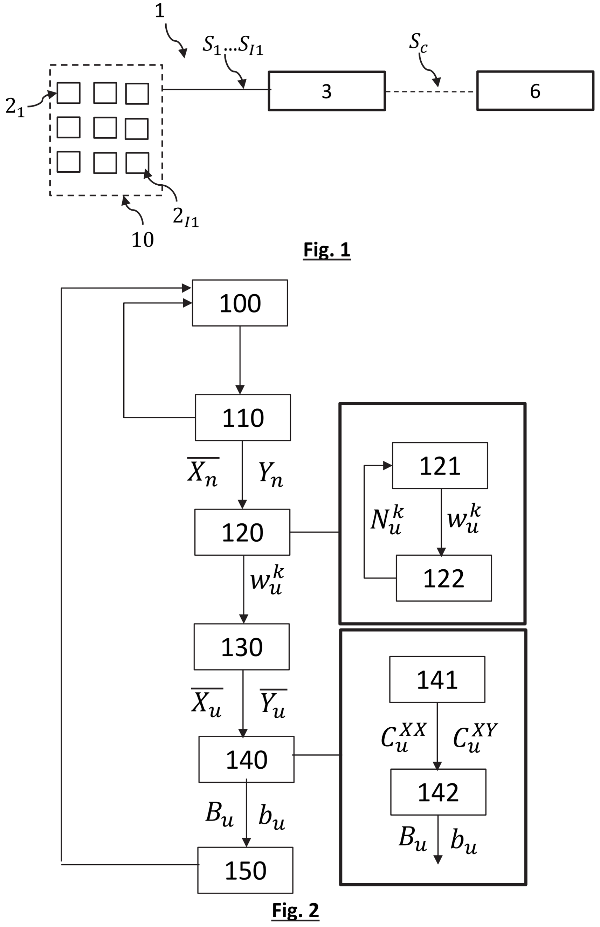

La figure 1 schématise une interface neuronale reliée à un utilisateur, et connectée à un processeur apte à mettre en œuvre un procédé selon l'invention.

La figure 2 représente les principales étapes d'un procédé de mise en œuvre de l'invention.

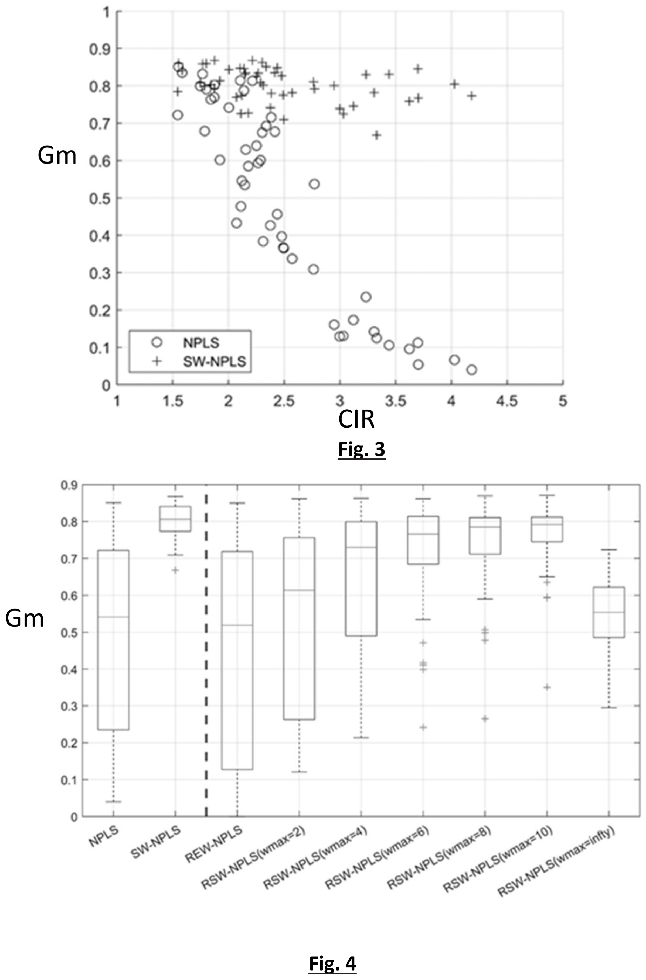

La figure 3 montre une moyenne géométrique du rappel de classes déterminée par un modèle prédictif hors ligne paramétré NPLS ou par SW-NPLS, en fonction d'un ratio de déséquilibre de classes. L'axe des ordonnées correspond à la moyenne géométrique et l'axe des abscisses correspond au ratio de déséquilibre des classes.

La figure 4 montre une comparaison de la moyenne géométrique du rappel de classes déterminée par un modèle prédictif en mettant en œuvre différents algorithmes d'apprentissage. L'axe des ordonnées correspond à la moyenne géométrique et l'axe des abscisses correspond à chaque algorithme d'apprentissage.

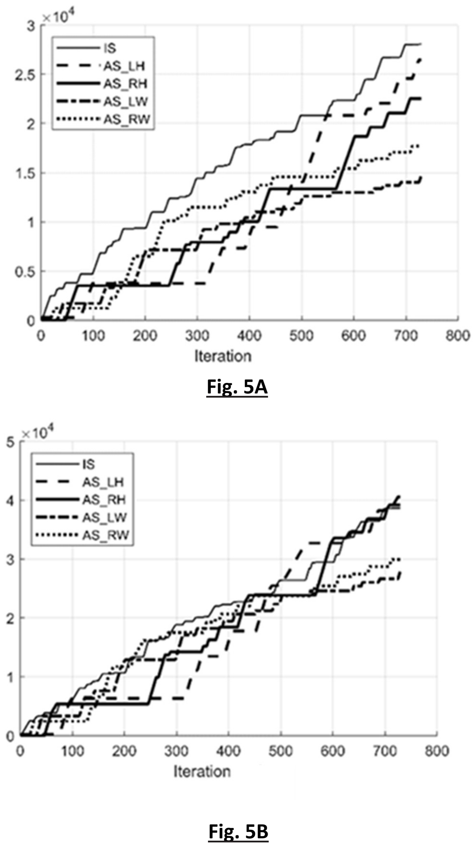

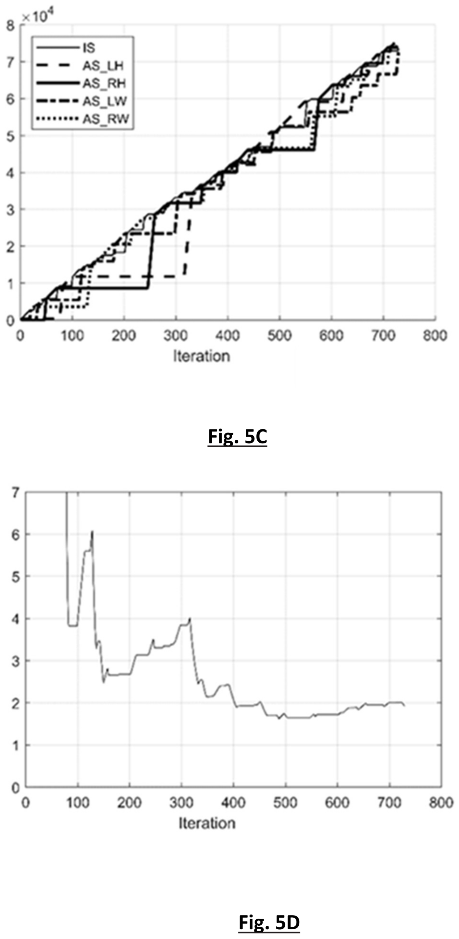

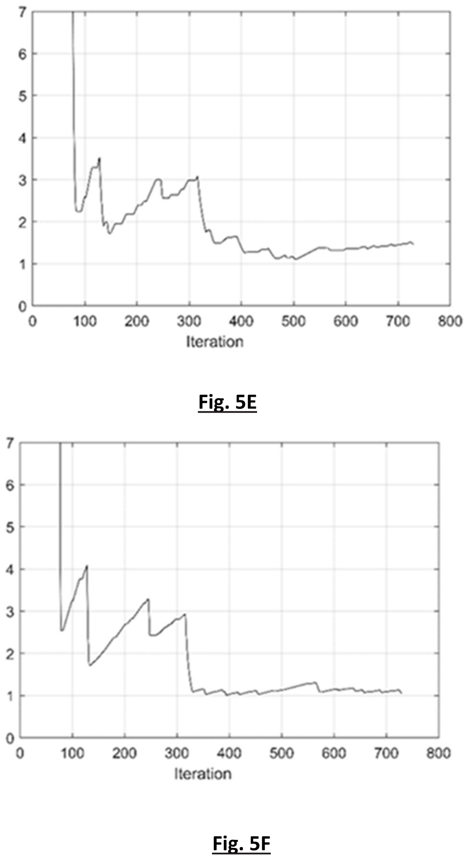

Les figures 5A à 5C représentent une évolution de la taille de classes en fonction d'un nombre d'itérations en mettant en œuvre un modèle prédictif entraîné respectivement par REW-NPLS (ce qui correspond à l'art antérieur), ainsi qu'en mettant en œuvre l'invention, en prenant en compte un poids maximal par classe égal à 2 et 10.

Les figures 5D à 5F représentent le ratio de déséquilibre de classes en fonction d'un nombre d'itérations en mettant en œuvre un modèle prédictif entraîné respectivement par REW-NPLS (ce qui correspond à l'art antérieur), ainsi qu'en mettant en œuvre l'invention, en prenant en compte un poids maximal par classe égal à 2 et 10.

## EXPOSE DE MODES DE REALISATION PARTICULIER

La figure 1 représente les principaux éléments d'une interface neuronale 1 selon l'invention. Il s'agit d'un dispositif comportant des capteurs $2_1...2_{I1}$, permettant l'acquisition de signaux électrophysiologiques représentatifs d'une activité neuronale. I1 est un entier correspondant au nombre de capteurs. Les capteurs $2_1...2_{I1}$ sont par exemple des électrodes corticales, l'indice E désignant le nombre d'électrodes corticales. Les capteurs $2_1...2_{I1}$ sont reliés à une unité de traitement 3, par exemple un microprocesseur, par une liaison filaire ou sans fil. Chaque capteur $2_1...2_{I1}$ est configuré pour détecter un signal électrophysiologique émis par un utilisateur 10. A partir de chaque signal électrophysiologique détecté, chaque capteur $2_1...2_{I1}$ transmet un signal électronique $S_1...S_{I1}$ à l'unité de traitement. L'unité de traitement 3 est apte à mettre en œuvre des algorithmes, de type modèle prédictif, pour détecter des caractéristiques des signaux électroniques $S_1...S_{I1}$ spécifiques à une tâche effectuée par l'utilisateur. L'unité de traitement 3 peut par exemple être un processeur relié à une mémoire mettant en œuvre des instructions permettant d'effectuer des algorithmes de décodage tels que décrits dans les publications citées en lien avec l'art antérieur. Ces derniers permettent de décoder les signaux physiologiques détectés de façon à déterminer les caractéristiques des signaux électrophysiologiques corrélées des tâches mentales effectuées par l'utilisateur 10.

Par tâche mentale, désignée par la suite tâche, on entend une action imaginée par un utilisateur auquel l'interface neuronale directe est raccordée. Il s'agit d'une action correspondant à une intention d'effectuer une tâche spécifique, en particulier une tâche motrice. La tâche spécifique est instruite à l'utilisateur par une tierce personne ou par un algorithme dédié.

Lors du fonctionnement opérationnel de l'interface neuronale directe 1, comme mentionné en relation avec l'art antérieur, l'utilisateur réalise successivement des tâches mentales. L'unité de traitement 3 reçoit les signaux électroniques $S_1...S_{I1}$ transmis par les capteurs $2_1...2_{I1}$, représentatifs des signaux électrophysiologiques produits par l'utilisateur et détectés par les capteurs. A partir des signaux électroniques détectés, lorsqu'une corrélation avec une tâche est détectée, le microprocesseur génère un signal de commande $S_c$ à l'attention d'un actionneur 6. Ainsi, l'interface neuronale directe décode les signaux électrophysiologiques produits par l'utilisateur 10 de façon à générer, à l'aide d'un modèle prédictif, des signaux de commande à un actionneur. La qualité du décodage est d'autant meilleure que l'algorithme de décodage a fait l'objet d'un apprentissage de qualité.

Au cours de l'apprentissage, l'utilisateur dispose d'une liste $T$ de tâches $T_k$ à effectuer. Comme décrit en lien avec l'art antérieur, au cours d'une phase d'apprentissage, un superviseur, humain ou machine, peut demander à l'utilisateur d'effectuer successivement des tâches $k$ choisies parmi la liste des K tâches. L'objectif est de déterminer progressivement les caractéristiques électrophysiologiques les mieux corrélées avec les tâches. Ces caractéristiques permettent ensuite d'établir le modèle prédictif, mis en œuvre lors du décodage, par lequel l'utilisateur 10 peut piloter l'actionneur 6 relié à l'unité de traitement 3.

Chaque tâche est à effectuer au cours d'une plage temporelle, dite époque, n. Le nombre d'époques à considérer pour l'apprentissage est très élevé, pouvant atteindre plusieurs centaines ou plusieurs milliers. Dans EP3563218, cité dans l'art antérieur, on décrit les principes d'un apprentissage par REW-NPLS. Selon un tel apprentissage, on dispose de données d'observation formant un tenseur tridimensionnel à chaque époque n.

Les signaux électrophysiologiques enregistrés font l'objet d'un prétraitement, selon lequel le signal de chaque électrode, durant chaque époque, fait l'objet d'une analyse fréquentielle. Il peut par exemple s'agir d'une transformée en ondelettes, par exemple une transformée en ondelette de Morlet, ou une décomposition CCWT (Continous Complex Wavelet Transform). La durée de chaque époque n peut être de 1 seconde ou 2 secondes, avec un recouvrement temporel entre deux époques consécutives. Plus précisément, durant chaque époque, une analyse fréquentielle est effectuée à intervalles réguliers, par exemple toutes les 100 ms. Une époque regroupe ainsi plusieurs analyses fréquentielles décalées dans le temps. Au cours d'une époque, plusieurs analyses fréquentielles sont effectuées, temporellement décalées les unes des autres.

A chaque époque n, on peut associer un tenseur d'observation $\overline{X_n}$, dont :

- le premier mode correspond à la position de chaque électrode, de dimension I1.
- le deuxième mode correspond aux positions temporelles des ondelettes, de dimension I2 ;
- le troisième mode correspond aux bandes de fréquences résultant de l'analyse fréquentielle, de dimension I3 ;

Une séquence d'apprentissage $u$ comporte $N$ époques n, s'étendant selon une plage temporelle $\delta t$. $u$ est un indice entier assigné chronologiquement à chaque séquence. A chaque séquence d'apprentissage correspond un tenseur d'apprentissage $\overline{X_u}$, de dimension NxI1xI2xI3 : le tenseur d'apprentissage $\overline{X_u}$ regroupe $N$ tenseurs d'observation $\overline{X_n}$ normalisés comme décrit par la suite. D'une façon plus générale, le tenseur d'apprentissage $\overline{X_u}$ est de dimension $N$xI1...x Ih x...IH, avec 1≤h≤H, h étant un indice et H étant un entier positif. Dans cet exemple, H = 3.

A chaque époque n correspond un signal de commande $Y_n$, qui peut être représenté par un vecteur de commande de dimension (K,1). Chaque terme du vecteur de commande correspond à une tâche $T_k$, parmi la liste $T$ des tâches prédéfinies. Au cours des $N$ époques formant la plage temporelle $u$, les différents signaux de commande forment une matrice $Y_u$ de dimension (K,N).

De façon alternative, le signal de commande $Y_n$ peut être une matrice, voire un tenseur multidimensionnel, auquel cas les différents signaux de commande forment un tenseur $\overline{Y_u}$ de mode $N$xJ1...x Jg x...JG. G ≥ 1. D'une façon générale, chaque tâche $k$ correspond à un signal de commande spécifique.

Lorsque l'interface est mise en œuvre, le modèle prédictif permet de passer du tenseur d'observation $\overline{X_n}$ à un signal de commande $Y_n$ pour chaque époque n.

Le modèle prédictif peut notamment être un modèle multilinéaire, appris par régression entre $\overline{X_u}$ et $Y_u$, par exemple par une méthode de type moindres carrés partiels multivariée. (NPLS). Un tel modèle permet une estimation du signal de commande selon une expression de type : $\hat{Y}_n = F(\overline{X_n})$ où $F$ est le modèle prédictif.

$$\hat{Y}_n = B \,\overline{X_n} + b + (1),$$

où

- B est un tenseur de prédiction, comportant des coefficients de prédiction ;
- b est un tenseur de biais ;

Le terme tenseur regroupe à la fois un vecteur (tenseur d'ordre 1), une matrice (tenseur d'ordre 2) ou des tenseurs d'ordre supérieur.

Dans l'exemple décrit par la suite, de façon non limitative, le modèle prédictif est tel que : $\hat{Y}_n = B X_n + b$ (1'), où $B$ est une matrice de dimension (K,P), et $X_n$ et $b$ sont des vecteurs de dimension (P, 1) et (K, 1). $X_n$ est un vecteur résultant d'une vectorisation du tenseur $\overline{X_n}$, avec $P = \prod_{h=1}^{H} I_h$

L'expression (1), peut être utilisée pour établir une probabilité d'émission. En prenant en compte des probabilités de changements d'états, l'état de l'utilisateur, en différents instants successifs, peut être estimé selon un modèle de Markov à états cachés, au cours duquel la tâche effectuée par l'utilisateur, en différents instants successifs, est assimilée à un état. En mettant en œuvre un algorithme, par exemple l'algorithme de type forward algorithm, on peut estimer les différents états successifs pris par l'utilisateur.

On va décrire, en lien avec la figure 2, les principales étapes d'un procédé permettant une mise en œuvre de l'invention, de façon à former un modèle prédictif tel que décrit par (1) ou (1'). Sachant que le modèle prédictif est élaboré en ligne, c'est-à-dire en temps réel ou quasi-temps réel, avec une mise à jour itérative, comme décrit dans EP3563218. Les étapes impliquant un traitement mathématique sont mises en œuvre par l'unité de traitement 3.

L'objectif du modèle prédictif est d'estimer, lors de l'utilisation de l'interface, à une époque n, le vecteur de commande $\hat{Y}_n$ selon (1) ou (1')

On sait que l'objectif d'un modèle établi par NPLS est de projeter le tenseur d'observation dans un espace latent de faible dimension, maximisant la covariance entre les tenseurs d'observation et de commande.

Dans EP3563218, on utilise les tenseurs $X_u$ et $Y_u$ et $C_{u-1}^{XX}$ et $C_{u-1}^{XY}$ déterminés au cours de séquences d'apprentissage consécutives $u$ et $u - 1$. On forme des tenseurs de covariances pondérés tels que :

$$C_u^{XX} = X_u^T X_u + \lambda C_{u-1}^{XX} \ (2)$$

et

$$C_u^{XY} = X_u^T Y_u + \lambda C_{u-1}^{XY} \ (3)$$

Où $\lambda$ est un facteur d'oubli. $\lambda$ est un réel positif, compris entre 0 et 1.

Cela permet d'établir un modèle prédictif, tel que décrit dans (1), en mettant en œuvre un algorithme récursif de type REW-NPLS. L'avantage est une mise à jour régulière du modèle prédictif, et un apprentissage effectué à partir de ressources mémoires limitées.

Cela permet de former un modèle prédictif tel que $\hat{Y}_n = B_u \overline{X_n} + b_u + E_n$. $B_u$ et $b_u$ sont réactualisés à chaque séquence.

Etape 100 : l'utilisateur imagine une tâche $k$, à un instant $t$, qui correspond à un signal de commande $Y_n$ connu. La tâche associée à l'instant $t$ peut notamment correspondre à un mouvement de l'actionneur, choisi parmi $K$ tâches possibles. Au même instant, on enregistre les signaux électrophysiologiques résultant des différents capteurs. Plus précisément, les signaux électrophysiologiques sont enregistrés au cours d'une époque, $n$, s'étendant selon une durée $\delta t$ à partir de l'instant $t$.

Chaque terme $Y_n(k)$ du signal de commande correspond à une tâche $k$. La valeur de $Y_n(k)$ est égale à 1 lorsque l'utilisateur imagine qu'il exécute la tâche $k$ et 0 autrement. Une des tâches peut être une tâche d'inactivité, désignée par l'acronyme IS (Inactive State)

Etape 110 : Pré-traitements. A chaque époque $n$, les signaux font l'objet d'une analyse temps-fréquence, comme décrit ci-avant, de façon à former un tenseur d'observation $\overline{X_n}$.

Les étapes 100 et 110 sont répétées $N$ fois, de façon à former un tenseur d'apprentissage $\overline{X_u}$. $N$ peut par exemple être égal à 150. $N$ est le nombre d'époques n formant la séquence d'apprentissage $u$. par la suite, $u$ désigne une séquence, c'est-à-dire une succession d'instants d'époques $n$. Par exemple, la durée totale des étapes 100 à 110 peut être de 15 secondes, chaque époque durant une durée $\delta t$ de 1 seconde, avec un décalage de 100 ms entre deux époques successives $n$, $n+1$, ce qui implique un recouvrement de 90% entre deux époques successives.

Etape 120 : Assignation d'un poids à chaque époque.

Les inventeurs ont constaté qu'un apprentissage récursif selon l'art antérieur, tel que décrit EP3563218 peut conduire à un déséquilibre de classes. Par déséquilibre des classes, on entend un déséquilibre de l'occurrence de certaines classes, correspondant respectivement à certains termes du vecteur de commande. En effet, certaines tâches, correspondant respectivement à certains termes du vecteur de commande, peuvent être sous-représentées, et nécessiter une durée d'apprentissage plus longue. Par exemple, lorsque l'actionneur est un exosquelette, la commande de la translation de la main peut nécessiter davantage d'entraînement que la commande de la rotation du poignet.

En outre, le déséquilibre affectant la difficulté d'apprentissage entre les tâches peut varier au cours du temps, entre différentes séquences d'apprentissage successives.

De plus, au cours de l'apprentissage, une tâche supplémentaire, peut être ajoutée, ce qui conduit à ajouter un terme au vecteur de commande.

Au cours de l'apprentissage, l'utilisateur, ou le superviseur, ne peut pas, par lui-même, compenser le déséquilibre entre les tâches, car le fait que l'apprentissage de certaines tâches $k$ soit plus difficile que d'autres ne peut pas être contrôlé par l'utilisateur.

Ainsi, à chaque époque n, est assigné un poids $w_n$, dont la valeur varie selon que l'on souhaite sur ou sous représenter l'observation à ladite époque n. Plus précisément, le poids $w_n$ dépend de la tâche $k$ assignée à l'époque n, parmi les $K$ tâches possibles. La tâche $k$ assignée à l'époque $n$, correspond au terme non nul du signal de commande $Y_n$. Au cours d'une même séquence $u$, les poids $w_n$ correspondant à une même tâche $k$, c'est-à-dire une même tâche, ont la même valeur. Ainsi, pour une même tâche $k$, le poids assigné, au cours d'une même séquence $u$ est $w_u^k$.

Sous-Etape 121 : Détermination des poids $\underline{w_n}$

Suite à une séquence $u$, les poids formant la matrice $diag(W_u)$ sont établis comme suit :

- on détermine le nombre d'occurrences $N_u^{maj}$ de la classe majoritaire suite à la séquence $u : N_u^{maj} = \max_k(\lambda N_{u-1}^k + n_u^k) \ (4)$, où : $N_{u-1}^k$ est le nombre d'occurrences de chaque classe $k$ suite à la séquence précédente $u$ - 1 .

Lors de la première séquence, $N_{u-1}^{k}$ est initialisé, par exemple égal à 0. $n_{u}^{k}$ est le nombre d'occurrences de chaque classe $k$, au cours de la séquence $u$, avant la pondération.

$\lambda$ est le facteur d'oubli précédemment décrit.

- on détermine le poids $w_{u}^{k}$ assigné à chaque classe $k$, durant la par

$$w_{u}^{k} = \frac{N_{u}^{maj} - \lambda N_{u-1}^{k}}{n_{u}^{k}} \text{ (5)}$$

et

$$w_{u}^{k} = 0 \text{ si } n_{u}^{k} = 0 \text{ (6)}$$

Il est préférable que des poids trop importants ne soient pas assignés à certaines tâches, de façon à ne pas augmenter le niveau de bruit affectant la détermination du modèle prédictif. Cela revient à éviter une surpondération de certaines classes $k$. Ainsi, on peut imposer qu'une valeur maximale $w_{max}$ soit établie . Lorsque (5) conduit à une valeur $w_{u}^{k}$ telle que $w_{u}^{k} \geq w_{max}$ , alors $w_{u}^{k} = w_{max}$ .

Après que le poids $w_{u}^{k}$ assigné à chaque classe $k$ a été défini, le poids $w_{n}$ associé à l'époque n est tel que $w_{n} = w_{u}^{k}$ , $k$ correspondant à la tâche associée à l'époque n.

<u>Sous-étape 122</u> : détermination de $N_{u}^{k}$

On détermine $N_{u}^{k}$ , qui correspond au nombre d'occurrences pondéré de la classe $k$, par :

$$N_{u}^{k} = \lambda N_{u-1}^{k} + w_{u}^{k} n_{u}^{k} \text{ (7)}$$

$N_{u}^{k}$ est destiné à être utilisé lors de la mise en œuvre des expressions (5) et (6) lors de la séquence suivante $u + 1$.

Outre la fréquence d'occurrences des tâches, d'autres critères peuvent être pris en compte pour assigner un poids à chaque époque $n$.

- la performance d'apprentissage : on peut par exemple sous-pondéré les tâches pour lesquels la performance d'apprentissage est considérée comme faible. La qualité d'apprentissage peut être évaluée par un indicateur de performance de type rappel (recall), qui correspond à un ratio entre le nombre d'occurrences de tâches correctement classées sur le nombre de tâches imaginées par l'utilisateur.
- la présence d'un outlier (valeur aberrante) à l'époque considérée , auquel cas le poids peut être choisi nul : il s'agit ici d'assigner un poids en fonction de la qualité des signaux enregistrés, de façon à minimiser ou annuler l'influence de signaux considérés comme aberrants;
- la survenue d'un changement de tâche, en sous-pondérant les instants survenant juste après un changement de tâche étant sous-pondérés par rapport aux instants postérieurs : il s'agit de prendre en compte un temps de réaction de l'utilisateur, survenant à chaque changement de tâche, et au cours duquel la réponse neurologique de l'utilisateur est considérée comme transitoire;

D'une façon plus générale, un critère de pondération est défini, pour chaque époque. Il peut s'agir d'un critère de fréquence d'occurrence de la tâche effectuée à chaque époque, ou de performance d'apprentissage de la tâche sélectionnée à chaque époque, ou un critère de qualité des signaux enregistrés à chaque époque, ou un critère temporel suite à un changement de tâche.

Les critères de pondération peuvent être combinés : par exemple, le poids d'une époque peut être défini à la fois en fonction de la fréquence d'occurrence et de la performance d'apprentissage.

Afin que l'invention ait un sens, il est préférable qu'au moins deux époques différentes d'une même séquence soient respectivement assignées de deux poids différents.

<u>Etape 130</u> : formation du tenseur d'apprentissage et du vecteur de commande pour la séquence $u$.

A chaque séquence d'apprentissage $u$ correspond un tenseur d'apprentissage $\overline{X}_{u}$, de dimension $N$xI1xI2xI3: le tenseur d'apprentissage $\overline{X}_{u}$ regroupe $N$ tenseurs d'observation $\overline{X}_{n}$ correspondant respectivement à $N$ époques $n$. Chaque

tenseur d'observation $\overline{X_n}$ est formé de termes ($x_{n,i1……iH}$), *où H est le nombre de modes du tenseur d'observation.*

La formation du tenseur d'apprentissage comporte une normalisation des tenseurs d'observation $\overline{X_n}$, puis un regroupement de chaque tenseur d'observation normalisé formé pour chaque époque *n* de la même séquence *u*.

Chaque tenseur d'observation $\overline{X_n}$ est normalisé par les opérations suivantes :

$$N_u^{Tot} = \lambda N_{u-1}^{Tot} + \sum_{n=1}^{N} w_n \quad (8)$$

- $N_u^{Tot}$ est un terme de normalisation pour la séquence *u* ; $N_u^{Tot}$ est la taille de la base d'apprentissage accumulé depuis le début de l'apprentissage en prenant en compte les poids.
- $\lambda$ est le facteur d'oubli précédemment décrit ;
- $w_n$ est le poids associé à chaque époque n de la séquence *u ;*
- $N_{u-1}^{Tot}$ est le terme de normalisation pour la séquence précédente *u* - 1. Lors de la première séquence (*u* = 1), on prend $N_0^{Tot} = 0$.

On calcule ensuite une moyenne $\mu_u^{Xi}$ pour chaque terme des *N* tenseurs d'observation, formant la séquence *u*

$$\mu_u^{Xi} = \frac{1}{N_u^{Tot}} (\lambda N_{u-1}^{Tot} \mu_{u-1}^{Xi} + \sum_{n=1}^{N} w_n x_{n,i}) (9)$$

avec *i* = (*i$_1$ …… i$_H$*)

$x_{n,i}$ est chaque terme de coordonnée *i* du tenseur d'observation $\overline{X_n}$ ;

On calcule ensuite une somme quadratique $SS_u^{Xi}$ :

$$SS_u^{Xi} = (\lambda SS_{u-1}^{Xi} + \sum_{n=1}^{N} w_n x_{n,i}^2)(10)$$

On calcule ensuite un écart type

$$\sigma_u^{Xi} = \sqrt{\frac{SS_u^{Xi} - N_u^{Tot} \mu_u^{i}{}^2}{N_u^{Tot} - 1}} \quad (11)$$

Et on normalise chaque terme du tenseur d'observation $\overline{X_n}$ par :

$$x_{n,i} \leftarrow \frac{x_{n,i} - \mu_u^{Xi}}{\sigma_u^{i}} \quad (12),$$

$\leftarrow$ signifie « est remplacé par »

On procède de la même façon pour chaque vecteur de commande $Y_n$. On calcule une moyenne $\mu_u^{Yk}$ pour chaque terme des *N* vecteurs de commande $Y_n$ pour la séquence *u* :

$$\mu_u^{Yk} = \frac{1}{N_u^{Tot}} (\lambda N_{u-1}^{Tot} \mu_{u-1}^{Yk} + \sum_{n=1}^{N} w_n y_{n,k}) (13)$$

$y_{n,k}$ est un terme de coordonnée *k* du vecteur de commande $Y_n$ ;

On calcule ensuite une somme quadratique $SS_u^{Yk}$ :

$$SS_u^{Yk} = (\lambda SS_{u-1}^{Yk} + \sum_{n=1}^{N} w_n y_{n,k}^2)(14)$$

On calcule ensuite un écart type

$$\sigma_u^{Yk} = \sqrt{\frac{SS_u^{Yk} - N_u^{Tot} \mu_u^{Yk^2}}{N_u^{Tot} - 1}} \ (15)$$

Et on normalise chaque terme du vecteur de commande par :

$$y_{n,k} \leftarrow \frac{y_{n,k} - \mu_u^{Yk}}{\sigma_u^{Yk}} \ (16)$$

L'étape 130 revient à normaliser chaque tenseur d'observation $\overline{X_n}$ et chaque tenseur de commande $Y_n$ en prenant en compte le poids $w_n$ associé à chaque époque $n$ de la séquence $u$. Il s'agit de calculer une moyenne temporelle et un écart type temporel, pondérés par le poids assigné à chaque époque, de chaque terme des tenseurs d'observation et du vecteur de commande. La moyenne temporelle et l'écart type temporel sont calculés pour les termes de mêmes coordonnées, en prenant en compte chaque époque n formant la séquence u.

On forme ensuite le tenseur d'apprentissage $\overline{X_u}$ et la matrice de commande $Y_u$ pour la séquence $u$. Chaque tenseur d'observation normalisé $\overline{X_n}$ peut être exprimé sous la forme d'un vecteur d'observation $X_n$, de dimension P, avec P = I1 x I2 x I3, suite à la vectorisation du tenseur $\overline{X_n}$, auquel cas le tenseur d'apprentissage $\overline{X_u}$ est une matrice d'apprentissage $X_u$ formée à partir des $N$ vecteurs d'observation : $X_u = (X_{n=1}, ... X_n=_N)^T$. La matrice d'apprentissage $X_u$ est de dimension ($N, P$).

On forme également une matrice de commande $Y_u$ à partir de chaque vecteur de commande normalisé. $Y_u = (Y_{n=1}, ... Y_{n=N})^T$. Dans cet exemple, $Y_u$ est de dimension ($N, K$).

Etape 140 : établissement du modèle prédictif.

On décrit à présent l'établissement d'un modèle prédictif à partir de la matrice d'apprentissage $X_u$ et de la matrice de commande $Y_u$ résultant de l'étape 130. Il s'agit tout d'abord d'établir le modèle prédictif décrit dans (1) et (1').

Sous-étape 141

A partir de la matrice d'apprentissage $X_u$ et de la matrice de commande $Y_u$, les matrices de covariance et de covariance croisées sont explicitées selon :

$$C_u^{XX} = X_u^T diag(W_u) X_u + \lambda C_{u-1}^{XX} \ (20)$$

et

$$C_u^{XY} = X_u^T diag(W_u) Y_u + \lambda C_{u-1}^{XY} \ (21)$$

$diag(W_u)$ est une matrice diagonale de dimension ($N,N$). Chaque terme de $diag(W_u)$ est le poids $w_n$ assigné à l'époque $n$, calculé lors de l'étape 120.

Sous-étape 142 : au cours de cette sous-étape, on détermine la matrice $B_u$ et le vecteur $b_u$ à partir des matrices de covariance et de covariance croisées $C_u^{XX}$ et $C_u^{XY}$ résultant de la sous-étape précédente, comme décrit dans EP3563218. Cela correspond notamment à l'étape 140 de EP3563218. Dans EP3563218, le modèle prédictif de commande est mis à jour par régression linéaire multivariée par moindres carrés partiels (NPLS), mais d'autres types de régressions multivariés peuvent être utilisables.

Le modèle prédictif peut être utilisé pour estimer la tâche la plus probable commandée par l'utilisateur, par un algorithme de type modèle de Markov caché (HMM), chaque tâche étant assimilée à un état de l'utilisateur. Dans ce cas, à chaque époque est assigné un état de l'utilisateur, l'état de l'utilisateur correspondant à une exécution d'une tâche. Les états successifs sont estimés par un algorithme de type HMM, en utilisant le modèle prédictif, comme décrit dans EP3789852.

Etape 150 : réitération. Les étapes 100 à 140 sont réitérées pour une séquence suivante, ce qui permet une mise à jour régulière, voire en continu, du modèle prédictif.

Essais expérimentaux

On a mis en œuvre la méthode précédemment décrite, sur la base des données collectées lors de l'essai clinique « Brain Computer Interface : Neuroprosthetic Control of a Motorized Exoskeleton », reporté dans clinicaltrials.gov sous la référence NCT02550522. Ces séquences sont décrites dans A. L. Benabid et al., "An exoskeleton controlled by an epidural wireless brain-machine interface in a tetraplegic patient: a proof-of-concept demonstration," The Lancet Neurology, vol. 18, no. 12, Art. no. 12, Dec. 2019, ainsi que dans A. Moly et al., "An adaptive closed-loop ECoG decoder for long-term and stable bimanual control of an exoskeleton by a tetraplegic," J. Neural Eng., vol. 19, no. 2, Art. no. 2, Mar. 2022.

Des signaux EcoG ont été enregistrés à l'aide d'un implant sans fil WIMAGINE tel que décrit dans Mestais C. et al « WIMAGINE : Wireless 64-Channel ECoG recording implant for long term clinical applications », IEEE Transactions on neural systems and rehabilitation engineering, Vol. 23, No1 , January 2015.

Un tenseur d'observation a été calculé toutes les 100 ms, selon une fenêtre glissante. L'analyse fréquentielle a été effectuée par transformée en ondelettes complexe continue (CCWT) sur la dernière seconde de signal, avec quinze ondelettes dérivées de l'ondelette mère de Morlet, et centrées sur quinze fréquences également espacées entre 10 et 150 Hz.

Chaque séquence était formée d'une mémoire tampon (buffer) stockant les données résultant de chaque capteur durant 15 secondes. Cela correspond à un jeu de 150 données d'observation par buffer. La durée d'une époque était de 1 seconde.

Au cours de l'apprentissage, des instructions étaient données à l'utilisateur d'effectuer des tâches mentales, destinées à contrôler un avatar virtuel. Le modèle prédictif a été entraîner pour décoder cinq tâches différentes :

- translation de la main droite (AS RH) ;
- translation de la main gauche (AS LH) ;
- rotation du poignet droit (AS RW) ;
- rotation du poignet gauche (AS LW) ;
- inactivité (IS).

A chaque époque, c'est-à-dire toutes les 100ms, on disposait d'un vecteur de commande $Yt$, formé de 5 termes $Yt(k)$, prenant la valeur 1 pour la tâche $k$ exécutée, $k$ étant compris entre 1 et 5.

On disposait de 10 séquences, parmi lesquels on a choisi aléatoirement 5 séquences pour l'apprentissage du modèle prédictif et 5 séquences pour les tests, au cours duquel le modèle prédictif a été appliqué. Cela a été répété 50 fois, en évitant d'avoir les mêmes jeux de séquences de test et de séquences d'apprentissage.

Les durées respectives des séquences étaient respectivement de 37 min 26 s, 29 min 8s, 48 min 3s, 9 min 35s, 50 min 6s, 53 min 2s, 31 min 41s, 37 min 58s, 47 min 34s, 22 min 12s.

Au cours des essais de test, chaque modèle a été évalué par un critère de performance correspondant à une moyenne géométrique du rappel, notée Gm. Le rappel d'une classe $k$ est défini par $P(\hat{Y}_t = k | Y_t = k)$ . La moyenne géométrique est définie comme :

$$\text{Gm} = \prod_{k=1}^{K} P(\hat{Y}_t = k | Y_t = k)^{1/K} \quad (22)$$

Gm est égal à 1 si l'ensemble des données sont bien classées. Les rappels calculés pour chaque classe, ainsi que la moyenne géométrique Gm, sont indépendants de la taille de chaque classe.

Pour chaque modèle prédictif, on a quantifié un ratio de déséquilibre entre les classes ou « class imbalance ratio », $CIR$ tel que

$$CIR = \frac{taille\ de\ la\ classe\ majoritaire}{taille\ de\ la\ classe\ minoritaire} \quad (23)$$

Plus le déséquilibre entre les classes est important, plus le ratio CIR est élevé. Un CIR égal à 1 correspond à un équilibre entre chaque classe.

Au cours des essais, $\hat{Y}_n$ a été estimé par l'application du modèle prédictif aux données correspondant à chaque époque, et $Y_n$ correspond au vecteur de commande correspondant à la tâche demandée.

On a pris en compte différentes valeurs $w_{max}$, respectivement comprises entre 2 et l'infini. Lorsque $w_{max}$ est égal à l'infini, cela signifie qu'il n'y a pas de valeur maximale assignée aux poids.

On a également effectué un apprentissage hors ligne, sans prise en compte du facteur d'oubli dans les matrices de covariance et de covariance croisée. Cela revient à définir le modèle prédictif :

- soit par NPLS ;
- soit en mettant en œuvre l'invention, sans prise en compte du facteur d'oubli ($\lambda = 0$) c'est-à-dire par NPLS pondérée, ou SW-NPLS (Sample Weighted NPLS - N moindres carrés partiels avec échantillons pondérés), auquel cas les tenseurs pris en compte, à chaque séquence, sont :

$$C_u^{XX} = X_u^T diag(W)X_u \ (24) \text{ et } C_u^{XY} = X_u^T diag(W)Y_u \ (24').$$

Dans ce cas, la matrice des poids *diag(W)* est une matrice diagonale de taille (*N,N*), dont chaque terme est associé à une époque n, et dont la valeur est le poids assigné à la classe *k* correspondant à l'époque *n*.

A chaque classe est assigné un poids $w_{u,}^k$, dont la valeur est

$$w_u^k = \frac{N_u^{maj}}{n_u^k} \ (25)$$

$N_u^{maj}$ correspond à la valeur de la classe majoritaire et $n_u^k$ correspond à la valeur de la classe *k*.

L'apprentissage hors ligne a été effectué en cumulant les séances utilisées pour l'apprentissage, la durée étant donc comprise entre 130 min et 240 min. Comme précédemment indiqué, l'apprentissage hors ligne est difficilement compatible avec une intégration dans un dispositif compact, car il requiert une mémoire importante compte tenu du nombre de données traitées. Il est utilisé ici en tant qu'outil de comparaison des apprentissages en ligne réalisés.

Sur la figure 3, on a représenté la moyenne géométrique Gm, telle que définie dans (9), calculée en prenant en compte les modèles prédictifs respectivement établis hors ligne par NPLS ou SW-NPLS. La moyenne géométrique est représentée en fonction du CIR. Chaque point de cette figure correspond à un test d'un modèle prédictif obtenu par NPLS ou par SW-NPLS en utilisant des données d'apprentissage correspondant à 5 sessions aléatoirement sélectionnées parmi les 10 premières sessions de la base de données. Le test du modèle prédictif a été effectué en utilisant les 5 autres sessions non sélectionnées pour l'apprentissage parmi les 10 premières sessions. Les expériences (apprentissage et test) ont été renouvelées 50 fois. On observe qu'en cas de déséquilibre important (ratio de déséquilibre élevé), la moyenne géométrique des rappels Gm de l'algorithme SW-NPLS surpasse largement celle obtenue par NPLS. Cela montre qu'en cas de déséquilibre entre les classes, le modèle prédictif obtenu par SW-NPLS est plus précis que le modèle prédictif obtenu par NPLS. Cette comparaison permet de confirmer l'intérêt d'une pondération des classes sous-représentées.

La figure 4 montre une comparaison de la moyenne géométrique du rappel de classes déterminées par différents algorithmes. Il s'agit d'une présentation de type boxplot (boite à moustaches). Les résultats étant représentés par une boite dont les bornes sont le premier et le troisième quartile, et le trait correspond à la médiane). Les valeurs extrêmes, à l'extérieur de la boite, montrent les valeurs minimales et maximales. L'axe des abscisses correspond à chaque algorithme mis en œuvre, avec, de gauche à droite :

- NPLS (hors ligne) ;
- SW-NPLS (hors ligne, avec pondération de chaque échantillon (Sample Weighted)) ;
- REW-NPLS (modèle récursif en ligne) ;
- l'invention (RSW-NPLS - modèle récursif en ligne, avec pondération de chaque échantillon), en considérant respectivement une valeur maximale $w_{max}$ égale à 2, 4, 6, 8, 10 et infinie. Le fait de considérer $w_{max}$ égal à l'infini revient à ne pas prendre en compte de valeur maximale.

Sur la figure 4, une ligne verticale en pointillés a été tracée, séparant les apprentissages hors ligne et en ligne.
On observe que la valeur maximale de Gm est obtenue en utilisant un modèle prédictif élaboré hors ligne par SW-NPLS. Cependant, comme précédemment indiqué, il s'agit d'un modèle dont l'apprentissage est effectué hors ligne. Cela semble correspondre à un optimum à atteindre. Les performances des modèles récursifs, dont l'apprentissage est effectué en ligne, sont meilleures lorsqu'une pondération des classes est mise en œuvre (modèles RSW-NPLS). On observe également que la valeur $w_{max}$ a une influence sur la performance de classification. En prenant en compte $w_{max}$ = 8 ou $w_{max}$ = 10, la performance de classification est proche de celle obtenue avec le modèle établi hors ligne par SW-NPLS (approche non récursive).
En l'absence de prise en compte d'une valeur $w_{max}$, c'est-à-dire en considérant $w_{max}$ égal à l'infini, les performances de classification sont moins bonnes qu'en prenant en compte une valeur $w_{max}$.
Les figures 5A à 5C représentent une évolution de la taille de classes en fonction d'un nombre d'itérations en mettant en œuvre un modèle prédictif entraîné respectivement par REW-NPLS (approche récursive par NPLS, sans pondération des époques, ce qui correspond à l'art antérieur), ainsi qu'en mettant en œuvre l'invention, en prenant en compte un poids maximal par classe respectivement égal à 2 et 10. Sans pondération (avec REW-NPLS), la classe IS est sur-représentée d'un facteur 2. Les algorithmes RSW-NPLS améliorent l'équilibre entre les classes. Lorsque $w_{max}$ = 2, seules deux tâches (SS$_{LH}$ et AS$_{RH}$) sont équilibrées avec la classe IS. Lorsque $w_{max}$ = 10, la contrainte imposée par l'algorithme est suffisamment importante pour que toutes les classes soient équilibrées au bout d'un nombre suffisant d'itérations.

Les figures 5D à 5F représentent le ratio de déséquilibre de classes en fonction d'un nombre d'itérations en mettant en œuvre un modèle prédictif entraîné respectivement par REW-NPLS (ce qui correspond à l'art antérieur), ainsi qu'en mettant en œuvre l'invention (RSW-NPLS), en prenant en compte un poids maximal par classe égal à 2 et 10. On observe que la pondération réduit le ratio de déséquilibre des classes, tel qu'explicité dans (8), et cela davantage lorsque $w_{max}$ = 10.

Sur les figures 5A à 5F, chaque courbe correspond à une tâche, parmi les tâches précédemment listées.

Variantes

Selon une possibilité, il est avantageux, durant l'apprentissage, de volontairement viser un déséquilibre spécifique entre les tâches effectuées par l'utilisateur. Cela peut notamment concerner une tâche prédéterminée, par exemple le repos. Un entraînement insuffisant de l'état de repos peut générer, lors de la mise en œuvre du modèle prédictif, des fausses activations, selon lesquelles l'utilisateur est considéré comme dans un état actif alors que l'état souhaité est un état de repos.

On peut par exemple viser une proportion d'occurrences plus élevée pour le repos que pour les autres tâches actives. Pour cela, pour chaque tâche $k$, on assigne une proportion cible $R_k$.

Dans la sous-étape 121, la proportion cible $R_k$ est prise en compte comme suit :

$$N_u^{maj} = \max_k \left( \frac{\lambda N_{u-1}^k + n_u^k}{R_k} \right) \ (4')$$

et

$$w_u^k = \frac{N_u^{maj} R_k - \lambda N_{u-1}^k}{n_u^k} \quad (5')$$

Les inventeurs estiment qu'il est préférable que la tâche de repos soit sur-représentée d'un facteur 2 à 2.5 par rapport aux autres tâches actives. Cela améliore la performance ainsi que la stabilité de la mise en œuvre du modèle direct.

Dans la description des sous étapes 121, on a calculé des poids $w_{u,k}$, correspondant respectivement à chaque classe, pour aboutir à un équilibre entre les différentes classes, de façon à surpondérer les classes d'autant plus qu'elles sont minoritaires. Selon une autre possibilité on peut sous-pondérer les classes majoritaires.

L'invention permet un apprentissage en ligne d'un modèle prédictif, et peut être mise en œuvre pour tout système de type BCI, y compris les dispositifs dans lesquels le décodage est transmis aux nerfs de la colonne vertébrale ou aux muscles. Dans ce cas, l'actionneur est préalablement implanté dans le corps de l'utilisateur : il s'agit par exemple d'un dispositif de stimulation.

**Revendications**

1. Procédé d'apprentissage d'une interface neuronale directe, l'interface neuronale directe étant reliée à des capteurs ($2_1...2_{I1}$) disposés autour du cerveau d'un utilisateur, chaque capteur étant configuré pour détecter un signal électrophysiologique dépendant d'une activité neuronale de l'utilisateur, l'interface étant configurée pour commander un actionneur (6) en fonction des signaux électrophysiologiques détectés, le procédé d'apprentissage comportant :

   - a) sélection d'une tâche mentale (k) à effectuer, choisie parmi une liste de tâches prédéterminée (1 ... k ... K) ;
   - b) exécution, par l'utilisateur, de la tâche mentale sélectionnée lors de l'étape a) et, durant l'exécution de la tâche :

      • acquisition des signaux électroniques, issus des différents capteurs ;
      • extraction de caractéristiques des signaux électroniques ;
      • formation d'un tenseur d'observation ($\overline{Xn}$) à partir des caractéristiques des signaux extraites ;

   - c) réitération des étapes a) et b) durant un nombre prédéterminé d'époques temporelles (n), formant une séquence (u);
   - d) formation d'un tenseur d'apprentissage ($\overline{X_u}$) à partir des tenseurs d'observation ($\overline{X_n}$) formés à chaque époque temporelle (n), et d'un tenseur de commande ($\overline{Y_u}$) à partir des tâches sélectionnées à chaque époque, chaque terme du tenseur d'apprentissage et du tenseur de commande étant associés à une époque de la séquence ;
- e) formation d'un modèle prédictif (F), par régression entre le tenseur d'apprentissage et le tenseur de commande, le modèle prédictif permettant d'estimer une tâche, choisie parmi la liste des tâches, imaginée par l'utilisateur en fonction du tenseur d'observation formé à chaque époque ;
- les étapes b), d) et e) étant mises en œuvre par une unité de traitement ;
le procédé étant **caractérisé en ce qu'**il comporte :

- définition d'un critère de pondération pour chaque époque ;
- assignation d'un poids($w_n$) à chaque époque, le poids étant défini en fonction du critère de pondération pour ladite époque, en fonction duquel à deux époques différentes, de la séquence, pour lesquelles le critère de pondération est différent sont assignés deux poids différents ;
le procédé étant tel que la formation du modèle direct est effectuée en fonction du poids respectivement assigné à chaque époque.

2. Procédé selon la revendication 1, tel que le poids assigné à une époque dépend de la tâche sélectionnée durant ladite époque.

3. Procédé selon l'une quelconque des revendications précédentes, dans lequel :

- les étapes a) à e) sont mises en œuvre durant plusieurs séquences successives ;
- le critère de pondération est défini en fonction d'une fréquence d'occurrence de chaque tâche, le poids de chaque époque ($w_n$) est d'autant plus élevé que le nombre d'occurrences de la tâche, suite aux séquences successives effectuées, est faible.

4. Procédé selon la revendication 3 comportant, après chaque nouvelle séquence (u), une mise à jour d'un nombre total d'occurrences pondéré pour chaque tâche ($N_u^k$), la mise à jour comportant, pour chaque tâche (k) :

- détermination d'un nombre d'occurrences ($n_u^k$) dans la nouvelle séquence (u) ;

- pondération du nombre d'occurrences, dans la nouvelle séquence, par le poids ($w_u^k$) respectivement assigné à la tâche dans la nouvelle séquence ;
- sommation du nombre d'occurrences pondéré de la tâche, dans la nouvelle séquence, au nombre total pondéré pour ladite tâche ($N_{u-1}^k$) résultant de la séquence précédente ($N_{u-1}^k$), ce dernier étant multiplié par un facteur d'oubli ($\lambda$).

5. Procédé selon l'une quelconque des revendications précédentes, dans lequel le critère de pondération est défini en fonction d'une performance d'apprentissage, le procédé comportant :

- détermination d'un indicateur de performance d'apprentissage de chaque tâche suite à chaque époque ;
- détermination du poids de chaque tâche en fonction du critère de performance d'apprentissage de la tâche.

6. Procédé selon l'une quelconque des revendications précédentes, dans lequel le critère de pondération est défini en fonction d'une qualité des signaux collectés à chaque séquence, le procédé comportant :

- détermination d'un critère de qualité des signaux collectés à chaque séquence ;
- détermination du poids de chaque tâche en fonction du critère de qualité des signaux des signaux collectés respectivement durant l'exécution de chaque tâche.

7. Procédé selon l'une quelconque des revendications précédentes, dans lequel dans l'étape e), le modèle prédictif est formé par régression multivariée, comportant un calcul d'un tenseur de covariance croisée entre le tenseur d'apprentissage et le tenseur de commande, le tenseur de covariance croisé ($C_u^{XY}$) de chaque séquence étant établi à partir d'un produit :

- du tenseur d'observation;
- du tenseur de commande;

- des poids ($w_n$) respectivement assignés à chaque époque.

8. Procédé selon la revendication 7, dans lequel

- l'étape c) est répétée de façon à former plusieurs séquences successives, à chaque séquence étant assigné un rang ($u$) ;
- l'étape d) est mise en œuvre pour chaque séquence ;
- lors de l'étape e), le modèle prédictif est formé à partir de deux séquences successives, à partir d'une somme du tenseur de covariance croisée établi pour la séquence de rang supérieur (u) et du tenseur de covariance croisée établi pour la séquence de rang inférieur (u-1) multiplié par un facteur d'oubli ($\lambda$).

9. Procédé selon l'une quelconque des revendications 7 ou 8, dans lequel :

- le tenseur d'apprentissage et le tenseur de commande sont formés d'une matrice, dont une dimension est le nombre d'époques dans la séquence ;
- les poids ($w_n$) forment une matrice diagonale ($diag(W_u)$), dont chaque dimension est le nombre d'époques par séquence, chaque terme de la matrice diagonale correspondant au poids assigné à chaque époque respectivement exécutée lors de ladite séquence.

10. Procédé selon l'une quelconque des revendications précédentes, dans lequel pour au moins une tâche spécifique, le poids est déterminé de façon que le nombre d'occurrences de ladite tâche spécifique, pondéré par le poids assigné à la tâche spécifique, soit supérieur au nombre d'occurrence d'au moins une autre tâche, pondéré par le poids assigné à ladite autre tâche.

11. Procédé selon l'une quelconque des revendications précédentes, dans lequel le poids assigné à chaque tâche est borné par une valeur maximale prédéfinie.

12. Interface neuronale directe, l'interface neuronale directe comportant des capteurs ($2_1...2_{I1}$) configurés pour être disposés autour du cerveau d'un utilisateur, et pour détecter des signaux électrophysiologiques, représentatifs d'une activité neuronale de l'utilisateur, l'interface étant configurée pour commander un actionneur (6), en mettant en œuvre un modèle prédictif de commande, le modèle prédictif étant configuré pour générer un signal de commande de l'actionneur à partir de signaux électrophysiologiques détectés ; l'interface comportant une unité de traitement (3), configurée pour acquérir les signaux électroniques lors de chaque étape b), et pour mettre en œuvre les étapes d) et e) d'un procédé selon l'une quelconque des revendications précédentes.

13. Interface selon la revendication 12, dans lequel l'actionneur est un dispositif externe à l'utilisateur ou un dispositif implanté dans le corps de l'utilisateur

**Fig. 1**

**Fig. 2**

**Fig. 3**

**Fig. 4**

**Fig. 5A**

**Fig. 5B**

**Fig. 5C**

**Fig. 5D**

**Fig. 5E**

**Fig. 5F**

Europäisches
Patentamt

European
Patent Office

Office européen
des brevets

**RAPPORT DE RECHERCHE EUROPEENNE**

Numéro de la demande

EP 25 22 7309

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | US 2023/248317 A1 (KIM SEONG EUN [KR]) 10 août 2023 (2023-08-10) * alinéas [0051] - [0058], [0070], [0146], [0151]; figure 13 * ----- | 1-13 | INV. G06F3/01 A61B5/369 A61B5/372 A61B5/00 A61B5/37 |
| A | SADIQ MUHAMMAD TARIQ ET AL: "A Comprehensive Approach for Enhancing Motor Imagery EEG Classification in BCI's", 11 octobre 2023 (2023-10-11), HEALTH INFORMATION SCIENCE; [LECTURE NOTES IN COMPUTER SCIENCE; LECT.NOTES COMPUTER], SPRINGER NATURE SINGAPORE, SINGAPORE, PAGE(S) 247 - 260, XP047672406, ISSN: 0302-9743 ISBN: 978-981-99-7107-7 [extrait le 2023-10-11] * le document en entier * ----- | 1-13 | |
| A | MOLY ALEXANDRE ET AL: "An adaptive closed-loop ECoG decoder for long-term and stable bimanual control of an exoskeleton by a tetraplegic", JOURNAL OF NEURAL ENGINEERING, IOP PUBLISHING, BRISTOL, GB, vol. 19, no. 2, 30 mars 2022 (2022-03-30), XP020419604, ISSN: 1741-2560, DOI: 10.1088/1741-2552/AC59A0 [extrait le 2022-03-30] * le document en entier * ----- | 1-13 | DOMAINES TECHNIQUES RECHERCHES (IPC) G06F A61B |

Le présent rapport a été établi pour toutes les revendications

| Lieu de la recherche | Date d'achèvement de la recherche | Examinateur |
|---|---|---|
| Munich | 17 avril 2026 | Fournier, Nicolas |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.................................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 25 22 7309

| Document brevet cité<br>au rapport de recherche | Date de<br>publication | Membre(s) de la<br>famille de brevet(s) | Date de<br>publication |
|---|---|---|---|
| US 2023248317 A1 | 10-08-2023 | AUCUN | |

## RÉFÉRENCES CITÉES DANS LA DESCRIPTION

**Documents brevets cités dans la description**

- US 9480583 B
- EP 3563218 A
- EP 3789852 A

**Littérature non-brevet citée dans la description**

- **ELISEYEV A** ; **AKSENOVA T**. Recursive N-way Partial Least Squares for Brain Computer Interface. *PIOS ONE*, July 2013, vol. 8 (7), e69962
- Brain-Computer Interface with cortical electrical activity recording. **YELISYEYEV A**. Human health and pathology. Université de Grenoble, 2011
- **A. L. BENABID et al.** An exoskeleton controlled by an epidural wireless brain-machine interface in a tetraplegic patient: a proof-of-concept demonstration. *The Lancet Neurology*, December 2019, vol. 18 (12)
- **A. MOLY et al.** An adaptive closed-loop ECoG decoder for long-term and stable bimanual control of an exoskeleton by a tetraplegic. *J. Neural Eng*, March 2022, vol. 19 (2)
- **MESTAIS C. et al.** WIMAGINE : Wireless 64-Channel ECoG recording implant for long term clinical applications. *IEEE Transactions on neural systems and rehabilitation engineering*, January 2015, vol. 23 (1)